# EUROPEAN PATENT APPLICATION

(11) **EP 4 427 801 A1**
(43) Date of publication of application: **11.09.2024**
(21) Application number: 24162189.5
(22) Date of filing: 07.03.2024
(51) Int. Cl.: A61N 1/40, A61N 2/02

(54) **APPARATUS FOR GENERATING A MAGNETIC FIELD**

(30) Priority: 07.03.2023 GB 202303315
(71) Applicant: Fluxmetrix Ltd, Leven KY9 1DU (GB)
(72) Inventor: CLARKE, Alan James, Stoke-on-Trent, ST8 7BB (GB)
(74) Representative: Hindles Limited

(57) **Abstract**

An apparatus configured to generate an oscillating magnetic field having a frequency of between 2 kilohertz and 1 megahertz in a target region, the target region defining a length direction. The apparatus comprises: a sheet conductor (7) extending in the length direction and around the target region to provide a wall of the target region surrounding at least 75% of a cross-section through the target region, the cross-section transverse to the length direction, and the sheet conductor having a first end separated from a second end thereof, the first end and the second end extending in the length direction and defining a separation region therebetween; and one or more capacitors (9) electrically connected to the sheet conductor at the first end and the second end, and across the separation region. The sheet conductor is configured to function as an inductor and, in combination with the one or more capacitors, to form a tank circuit, to thereby generate an oscillating magnetic field in the target region when the tank circuit is driven at a resonant frequency of the tank circuit.

## Description

### Field of the invention

The present invention relates to apparatus for generating a magnetic field, in particular to apparatus for use in magnetic hyperthermia treatments and thermal drug release systems. Typically, the invention relates to apparatus for use in internal magnetic hyperthermia for cancer treatment.

### Background to the invention

Hyperthermia therapy is a type of medical treatment in which body tissue is exposed to temperatures higher than the normal human body temperature to damage and kill cancer cells or to make cancer cells more sensitive to the effects of radiation and certain anti-cancer drugs. There is growing evidence to support its use as a primary treatment.

Hyperthermia treatment can be divided into two fields: external hyperthermia and internal hyperthermia treatments. In external hyperthermia treatment, the heat is applied from outside the body using various means such as microwaves, radiofrequencies, ultrasound etc. In internal hyperthermia treatment, certain foreign substances are inserted inside the body to function as sources of heat.

Since tumours are growing, they stimulate the body to create new blood vessels in and around them. Newly formed blood vessels are slightly porous, allowing particles of a certain size to pass through the pores from the blood, and accumulate in a tumour. In mature blood vessels, particles of this size will simply flow past.

Therefore, an intravenous injection including appropriately sized particles into the bloodstream allows the particles to find their own way into cancerous tissue and become concentrated through accretion.

In other examples, appropriately sized particles can be injected directly into the cancerous tumours to provide a sufficiently high concentration of nanoparticles within the cancerous tissue, compared with far fewer, if any, particles in the healthy tissue outside the cancerous tumours.

Magnetic hyperthermia is an internal hyperthermia form of experimental cancer treatment which uses various forms of magnetic nanoparticles subjected to an alternating magnetic field to produce heat. If magnetic nanoparticles are introduced into a tumour and the patient, or the part of the patient containing the magnetic particles, is placed in an alternating magnetic field of suitable amplitude and frequency, the tumour temperature will rise.

The use of internal magnetic hyperthermia for cancer treatment appears to be a most promising approach because of the superior temperature homogeneity that can be achieved.

An essential feature of this treatment is that all parts of the treatment target receive uniform particle excitation, whilst surrounding healthy tissue is not exposed to a hazardous level of magnetic field.

Traditional methods of homogeneous field generation include solenoid coils, Helmholtz coils and Maxwell coils. Each of these methods has limitations on what can be practically achieved in terms of electrical parameters such as voltage level or power requirement before a sufficient volume of the required field intensity is created.

Coils, such as solenoid coils, are unsuitable for the generation of large volumes of homogeneous field at frequencies of interest. As both the area enclosed by each turn of the coil and the number of turns is increased, there is a large increase in the inductance of this component.

To resonate this inductance at the required frequency, the requisite capacitance becomes small and the voltage necessary to drive the required current through the inductive reactance of the coil becomes prohibitively large.

Therefore, a significant issue of how to scale up the size of the equipment used exists. Typical laboratory hyperthermia research instruments have a limitation in the order of a cylinder of 50 mm diameter, by 75 mm length. This is too small to accommodate many parts of a patient's body.

US4402309 in the name of Harrison describes a circular magnetic electrode used for external hyperthermia treatment. Harrison describes an improvement to short wave diathermy equipment, which is traditionally used to treat muscles and joints, generally in the area of athletic activity. In particular, a short wave frequency of 13.56 MHz is used.

The magnetic electrode of Harrison comprises a cylinder used as an inductor and, to create a homogeneous field within the cylinder, capacitance layers of plate/dielectric are manufactured from the plates of the inductor.

The capacitor(s) permit a resonance within the inductor/capacitor circuit, boosting the circulating current within the circuit. However, an inherent limitation of the apparatus of Harrison is that significantly lower frequencies are not practically possible with the used capacitor arrangement.

Although the treatment of tumours is mentioned, the apparatus of Harrison is not suitable for surface or internal magnetic hyperthermia treatment. The injected magnetic nanoparticles would not efficiently mediate the transfer of energy from the magnetic field to the tissues when in a field generated by frequencies higher than 1 megahertz. Rather, for internal magnetic hyperthermia treatment, the optimum frequency is much lower, in the order of 10's or low 100's of kilohertz.

In order to work at significantly lower frequencies, In Harrison's design the capacitors would have to have an enormous and impractical plate area (to resonate a given inductance at one hundredth the original frequency would require ten thousand times more capacitance).

At the frequencies described by Harrison, eddy currents are induced in tissues, thus heating them. However, this causes the heating of healthy tissue as well as tumour tissue, and, in heating any tumour, damage will occur to healthy tissue as well.

In Harrison's design using the cylinder plates as capacitors at the frequencies and voltages required for the treatment of cancerous tumours would, for example, require the plates to be as large as a football pitch.

It is in this context that the present inventions have been devised.

### Summary of the invention

In accordance with an aspect of the present invention, there is provided an apparatus configured to generate an oscillating magnetic field having a frequency of between 2 kilohertz and 1 megahertz in a target region, the target region defining a length direction. The apparatus comprises: a sheet conductor extending in the length direction and around the target region to provide a wall of the target region surrounding at least 75% of a cross-section through the target region, the cross-section transverse to the length direction, and the sheet conductor having a first end separated from a second end thereof, the first end and the second end extending in the length direction and defining a separation region therebetween; and one or more capacitors electrically connected to the sheet conductor at the first end and the second end, and across the separation region. The sheet conductor is configured to function as an inductor and, in combination with the one or more capacitors, to form a tank circuit, to thereby generate an oscillating magnetic field in the target region when the tank circuit is driven at a resonant frequency of the tank circuit.

Thus, by use of the one or more capacitors connected in parallel to the sheet conductor, a relatively compact arrangement can be provided which can still generate a sufficiently strong oscillating magnetic field to cause heating by oscillation of ferromagnetic nanoparticles within a body provided in the target region. The present invention is particularly well suited for use in treatments of internal magnetic hyperthermia. As a result, the present invention can be used at relatively large scales, even, for example, where the target region contains the whole body of a human subject. Prior art solutions cannot be provided at this scale without being impractically bulky, and/or without suffering from regions of the target region having unacceptably high field strength, resulting in excessive heating of regions of the body provided within the target region, even where those regions include far fewer ferromagnetic nanoparticles.

It will be understood that a capacitor is an electrical component having an electrical insulator (a dielectric material) provided between two electrically conductive electrodes. In the present cases, the insulator will be a physical material (i.e. not air). A capacitor is used to store charge at one point which can be discharged at a later point.

The one or more capacitors may be one or more individual capacitors.

The generated oscillating magnetic field in the target region may be a substantially homogeneous oscillating magnetic field. In other words, a field strength of the oscillating magnetic field in a first portion of the target region is less than 20% different, such as less than 10% different from the field strength of the oscillating magnetic field in any other portion of the target region. A maximum variation in the rms magnetic field strength of the oscillating magnetic field in the target region may be less than 10%, such as less than 5%. Thus, substantially uniform heating can be experienced by the regions of the body having similar concentrations of nanoparticles during treatments of internal magnetic hyperthermia. Furthermore, areas of relatively high strength magnetic field might cause a damaging heating effect in healthy tissue, which can be avoided by use of a substantially homogeneous oscillating magnetic field.

The rms magnetic field strength of the oscillating magnetic field in the target region may be greater than 3 millitesla, such as greater than 5 millitesla, for example greater than 10 millitesla. The rms magnetic field strength of the oscillating magnetic field in the target region may be less than 50 millitesla, such as less than 30 millitesla, for example less than 20 millitesla. Thus, the heating effect is sufficient to cause damage to nanoparticle-doped cancerous tumours without damaging surrounding healthy tissue without the same levels of nanoparticles.

The sheet conductor may provide a wall surrounding at least 90% of the cross-section through the target region. In other words, a gap in the target region, not bounded by the sheet conductor is typically no more than 25% of the cross-section through the target region, such as no more than 10% of the cross-section through the target region. Thus, most of the target region is surrounded by the sheet conductor, at least in cross-sections defining planes transverse to the length direction. Typically, the sheet conductor provides a wall of the target region surrounding at least 75% of all cross-sections through the target region and transverse to the length direction, along the length of the target region.

The oscillating magnetic field configured to be generated by the apparatus may have a frequency less than 500 kilohertz in the target region. The oscillating magnetic field configured to be generated by the apparatus may have a frequency less than 300 kilohertz in the target region. The oscillating magnetic field configured to be generated by the apparatus may have a frequency greater than 50 kilohertz in the target region. The oscillating magnetic field configured to be generated by the apparatus may have a frequency greater than 100 kilohertz in the target region. The oscillating magnetic field configured to be generated by the apparatus may have a frequency between 100 kilohertz and 300 kilohertz in the target region. Thus, heating is caused by oscillation of nanoparticles within the body, rather than generation of eddy currents in the body, which would be the case at higher frequencies.

The wall may define a first internal surface normal, having a component directed away from the first end and the second end. The wall may further define a second internal surface normal, having a first component directed away from the first end and the second end, and a second component perpendicular to the first component and directed towards the first internal surface normal. In some examples, it may be that the shape of the sheet conductor is described using the features of this paragraph instead of the feature that the wall extends around at least 75% of the cross-section.

A distance between the first end and the second end across the separation region may be less than 10 centimetres. The distance may be less than five centimetres. Thus, larger target regions, the sheet conductor encloses most of the target region in the planes transverse to the length direction.

The first end may be connected to the second end via the sheet conductor in a first direction. The first end is separated from the second end by less than 10 centimetres in a second direction. The first end may be separated from the second end by less than 5 centimetres.

The sheet conductor may define an internal surface facing into the target region and an external surface opposite the internal surface. The one or more capacitors may be electrically connected to the sheet conductor at the internal surface at the first end and at the internal surface at the second end. Thus, the current flowing in the sheet conductor need only flow on or close to the internal surface of the sheet conductor to flow between the sheet conductor and the one or more capacitors, reducing resistance and ensuring the apparatus is particularly efficient.

The one or more capacitors may be a plurality of capacitors. The plurality of capacitors may be connected to the sheet conductor at the first end and the second end, at a plurality of different lengthwise locations. Thus, at a plurality of different lengthwise locations along the sheet conductor, each respective capacitor provides a capacitance in parallel across the two ends of the sheet conductor, helping to ensure a substantially homogeneous magnetic field is generated in the target region.

Each of the plurality of capacitors may be connected to the sheet conductor at a point less than 10 centimetres along the surface of the sheet conductor from where a further capacitor is connected thereto. Each of the plurality of capacitors may be connected to the sheet conductor at a point less than 5 centimetres along the surface of the sheet conductor from where a further capacitor is connected thereto. In some examples, it may be that at each point along the first end and the second end of the sheet conductor, at least one of the plurality of capacitors is provided within less than 10 centimetres, such as within less than 5 centimetres. It may be that the whole length of the gap between the first end and the second end of the sheet conductor is filled by the plurality of capacitors.

The plurality of capacitors may be at least ten capacitors, together connected to the sheet conductor at at least ten different lengthwise locations. The plurality of capacitors may be at least 20 capacitors. It may be that each of the plurality of capacitors is connected to the sheet conductor at a different lengthwise location. Each of the plurality of capacitors may have the same rated capacitance. In some examples, the capacitance of each of the plurality of capacitors may be within 10 percent of an average capacitance of the plurality of capacitors. Importantly, the sum of the capacitance of the plurality of capacitors arranged in parallel is configured to resonant the inductance of the sheet conductor at the desired operating frequency.

The one or more capacitors may be one or more high current capacitors. It will be understood that a high current capacitor is substantially any capacitor capable of carrying greater than 300 A rms. It may be that each of the high current capacitors are capable of carrying greater than 600 A rms.

The apparatus may be adapted to operate at a maximum current of greater than 10,000 A rms, preferably greater than 15,000 A rms.

The cross-section through the target region may define a substantially curved shape (e.g. for the sheet conductor), having a radius of curvature for any concave regions with respect to the target region of greater than five centimetres. In other words, the sheet conductor is shaped so as to avoid sharp concave corners. This helps promote the generation of a substantially homogeneous magnetic field in the target region. The cross-section may be substantially circular, or ovular. It will be understood that even with the gap between the first and second end, the cross-section will still be regarded as substantially circular or substantially ovular, providing the rest of the cross-section follows a generally circular or ovular path respectively.

The sheet conductor may comprise a first planar extension at the first end, extending away from the target region, and a second planar extension at the second end, extending parallel to and separated from the first planar extension. The separation region may be defined between the first planar extension and the second planar extension. The first planar extension may extend substantially the same distance as the second planar extension. The first planar extension and the second planar extension may extend away from the target region by a distance of more than two centimetres. The first planar extension and the second planar extension may extend away from the target region by a distance of less than 30 centimetres.

A minimum extent of the target region in a direction transverse to the length direction may be greater than 100 millimetres. A minimum extent of the target region in a direction transverse to the length direction may be greater than 200 millimetres. A minimum extent of the target region in a direction transverse to the length direction may be greater than 300 millimetres. A maximum extent of the target region in a direction transverse to the length direction may be less than two metres. The length of the target region may be greater than 100 millimetres. The length of the target region may be greater than 30 centimetres. The length of the target region may be greater than 50 centimetres. The length of the target region may be greater than 100 centimetres. The length of the target region may be less than two metres.

The maximum extent of the target region in the direction transverse to the length direction may be less than the length of the target region.

The length of the target region is typically less than the length of the sheet conductor. The sheet conductor may extend beyond the target region in the length direction by at least five percent of the length of the target region, such as at least ten percent of the length of the target region. The sheet conductor may extend beyond the target region in the length direction by at least 50 millimetres, such as at least 100 millimetres. The sheet conductor may extend beyond the target region in the length direction on each lengthwise side of the target region.

It will be understood that the target region is the volume throughout which the oscillating magnetic field having the required properties is generated (i.e. the magnetic field density can be considered substantially homogeneous, such as within 10% of a target magnetic field density).

The apparatus may be configured to generate the oscillating magnetic field in the target region having a substantially homogeneous field strength throughout the target region.

The apparatus may be configured to determine the resonant frequency at which to drive the tank circuit and to control the apparatus in dependence on the determined resonant frequency. It will be understood that the resonant frequency of the apparatus is typically dependent on one or more of an inductance of the sheet conductor, a capacitance of the one or more capacitors, a resistance of the circuit components of the apparatus, including the sheet conductor and the one or more capacitors. The resonant frequency of the apparatus may be dependent on the composition of any body included in the target region.

The apparatus may be configured to, when organic tissue is provided in the target region, the organic tissue including nanoparticles comprising a ferromagnetic material, cause heating of the organic tissue in the regions having high concentrations of the nanoparticles, in response to providing the alternating current supply signal to the sheet conductor. Thus, where nanoparticles are included predominantly within cancerous tumours in the organic tissue, the apparatus can be used to cause substantially uniform heating of the cancerous tumours to a level to cause damage of the cancerous tumours without causing significant damage to healthy tissue nearby.

The nanoparticles may have a maximum extent of between 5 nanometres and 25 nanometres. Thus, the apparatus will typically work to cause heating when nanoparticles of such sizes are provided in organic tissue within the target region.

The apparatus may further comprise a cooler for cooling at least one of the one or more capacitors and the sheet conductor. It will be understood that the significant energy dissipation by the sheet conductor and the one or more capacitors will typically require a cooling component. The cooler may define a plurality of conduits in a housing of the apparatus. The plurality of conduits are typically routed close to a surface of the sheet conductor, and/or close to the one or more capacitors. Thus, cooling fluid channelled through the plurality of conduits can act to cool the sheet conductor and/or the one or more capacitors.

The apparatus may further comprise a magnetically permeable portion, having a magnetic permeability greater than air. The magnetically permeable portion may be provided to cover each lengthwise end portion of the sheet conductor. The magnetically permeable portion may comprise a return portion running parallel to the sheet conductor in the length direction, and connected to the portions of the magnetically permeable portion covering each lengthwise end portion of the sheet conductor. Thus, a total amount of electrical power required to generate a given RF magnetic field density is decreased compared to the apparatus in the absence of the magnetically permeable portion. The magnetically permeable portion may be formed from a low-loss grade of ferrite..

The sheet conductor may comprise copper. The sheet conductor may have at least a surface region formed from an electrically conductive material. The surface region of the sheet conductor may comprise graphene.

The apparatus may further comprise a temperature sensor configured to output a temperature signal indicative of a temperature within the target region. Thus, the temperature within the target region can be monitored. It may be that the temperature sensor is arranged to measure an air temperature within the target region. In other examples, the temperature sensor is arranged to measure an internal temperature of a body inserted within the target region. The temperature of the body inserted within the target region may be monitored using one or more fibre-optic thermometers.

The apparatus may further comprise a controller. The controller may be configured to control at least one of a frequency and an amplitude of a supply signal for driving the tank circuit. Thus, the controller causes control of at least one of a magnetic field strength and a temperature within the target region. In one example, the controller may be configured to control the frequency and the amplitude of the supply signal.

The apparatus may comprise a signal generator configured to output a supply signal having an amplitude and a frequency for driving the tank circuit.

The apparatus may further comprise a power supply to provide power to the signal generator, and/or one or more amplifiers to amplify the signal generated by the signal generator.

The apparatus may include a variable high-power excitation amplifier for adjusting (e.g. increasing) the amplitude of the supply signal.

The frequency of the supply signal may be adjustable. The frequency of the supply signal may be adjustable to maintain optimum resonance.

The controller may be configured to analyse a waveform of the tank circuit formed by the sheet conductor and the one or more capacitors and determine a level of harmonic distortion within the waveform.

The controller may be configured to perform a mathematical calculation on the waveform to determine a value of total harmonic distortion (THD).

The controller may be configured to perform a Fast Fourier Transform (FFT) on the waveform to determine a value of specific harmonics of the operating frequency, or of the total harmonic distortion (THD).

The controller may be configured to adjust the frequency of the supply signal to reduce the determined level of harmonic distortion within the waveform.

The controller may be configured to provide closed loop control of the adjustment of the frequency of the supply signal.

The controller may be configured to determine a value of the field intensity of the oscillating magnetic field in the target region. The field intensity may be determined using the geometry of the sheet conductor and the value of the current flowing through the sheet conductor.

The value of the current flowing through the cylinder may be determined from the ratio of the voltage across the sheet conductor and the impedance of the sheet conductor.

The apparatus may comprise a sensor device for sensing the intensity of the generated magnetic field. The controller may be configured to determine the value of the field intensity in dependence on an output from the sensor device for sensing the intensity of the generated magnetic field.

The controller may be configured to determine an optimal value of the field intensity.

The controller may be configured to adjust the power and/or the gain of the amplifier to modify the field intensity towards the optimal value of the field intensity.

The controller may be configured to regulate heating in dependence on the output from the temperature sensor, using the amplifier.

The controller may be configured to provide closed loop control of the adjustment of the field intensity.

The controller may be configured to provide closed loop control of the temperature of the body containing the nanoparticles, provided in the target region.

The controller itself is believed to be novel and so, in accordance with a still further aspect of the present invention, there is provided a controller for apparatus for use in internal magnetic hyperthermia, the apparatus as described hereinbefore, the controller configured to: generate a signal to cause resonance of the sheet conductor; analyse a waveform of a tank circuit of the apparatus, including the sheet conductor; determine a level of harmonic distortion within the waveform; and adjust the generated signal to reduce the determined level of harmonic distortion within the waveform.

The controller may further comprise any of the features described hereinbefore with reference to the apparatus itself.

Viewed from another aspect, the present invention extends to a method of heating a body. The method comprises: providing the body within the target region of an apparatus according to any preceding claim; and when the body is within the target region, operating the apparatus to generate an oscillating magnetic field in the target region to thereby heat one or more portions of the body having nanoparticles therein, the nanoparticles comprising a ferromagnetic material.

The method may further comprise supplying the nanoparticles to the body prior to providing the body within the target region. The method may further comprise performing substantially any of the functions described hereinbefore with reference to the controller.

The apparatus described hereinbefore can sometimes be considered as suitable for use in internal magnetic hyperthermia.

According to another aspect of the present invention there is provided an apparatus for use in internal magnetic hyperthermia, the apparatus comprising: an inductor device comprising a single turn elliptical or extended elliptical body in the form of a tube; the inductor having a longitudinal slot.

The sheet conductor may sometimes be referred to as the inductor device described herein, and any features described with reference to the inductor device will be understood to be applicable to the sheet conductor described elsewhere.

The elliptical aspect of the inductor tube part of this invention is designed to reduce the amount of voltage and current necessary to produce an homogeneous field of the volume required in treating human bodies.

In a further aspect the inductor may be an oval tube.

In a yet further aspect the inductor may be a circular or quasi-circular tube

A plurality of capacitors is provided at or near the slot, wherein the inductor device and the capacitors are connected in parallel to form a resonant circuit and are adapted to resonate the inductor device at a chosen frequency, thereby producing a volume of homogeneous magnetic field within the body, and wherein the capacitors are high current capacitors and wherein the predetermined frequency is in the range of kilohertz.

The smallest dimension of the elliptical cylinder may be such as to be smaller than 200mm or may be scaled up to receive a human torso.

The length of the cylinder may be greater than 100 mm. The length of the cylinder may be greater than 1,000 mm.

The inductor device and the high current capacitors may be connected in parallel.

The slot may define two opposing free ends of the cylindrical body.

A flange may be provided at each free end, each of the two flanges having a planar surface which are spaced from and parallel to each other.

The high current capacitors may be provided at or near the flanges.

The capacitors may be placed equidistantly along the slot

The apparatus may include cooling means. The tube members may define one or more flow paths for a coolant to flow through the cylinder. The coolant may be water, or other cooling fluid.

The cylinder may comprise a copper material; other low resistance materials may be used to lower the voltage and current requirements of the higher volume apparatus.

The aspect ratio of the cylinder, defined as the ratio of the diameter of the cylinder to the length of the cylinder, may be between 0.5 and 5. The aspect ratio of the cylinder may be between 1 and 3.

The high current capacitors may be arranged side-by-side along the slot. Each of the high current capacitors may be equally spaced.

The cylinder may comprise one or more tube members. The tube members may be provided as a plurality of annular rings which are conductively bonded together to form the cylinder.

The coolant may be provided at a pressure greater than atmospheric pressure.
The connected annular rings may define the one or more flow paths. A manifold may be provided for directing the coolant from a source through the plurality of flow paths. The cooling means may be adapted to cool the capacitors.

Alternatively, the cylinder may be formed from copper, or copper sheet plated with silver, or graphene film, or a super-conducting film, or other suitable material.

The apparatus may be used to excite a plurality of magnetic particles (not part of this invention) which are insertable into the body of a patient. The magnetic particles may comprise magnetic nanoparticles. The magnetic nanoparticles may be adapted to produce heat when within the volume of homogeneous alternating magnetic field.

The apparatus may include a permeable material. The permeable material may be adapted to reduce the magnetic reluctance of the air path external to the inductor device.

### Description of the Drawings

An example embodiment of the present invention will now be illustrated with reference to the following Figures in which:
Figure 1 is a representation of a cancerous tumour;
Figure 2 illustrates infusion of the tumour of Figure 1 with a nanoparticle infused suspension;
Figure 3 illustrates the response of nanoparticles to an oscillating magnetic field;
Figure 4 shows an inductor component in accordance with an embodiment of the present invention;
Figure 5 shows a schematic indication of apparatus in accordance with an embodiment of the present invention;
Figures 6A and 6B show example cooling systems for use in an embodiment of the present invention;
Figure 7 shows a schematic circuit diagram for a control system in accordance with an aspect of the present invention; and
Figure 8 shows an example of apparatus in accordance with an embodiment of the present invention.

### Detailed Description of an Example Embodiment

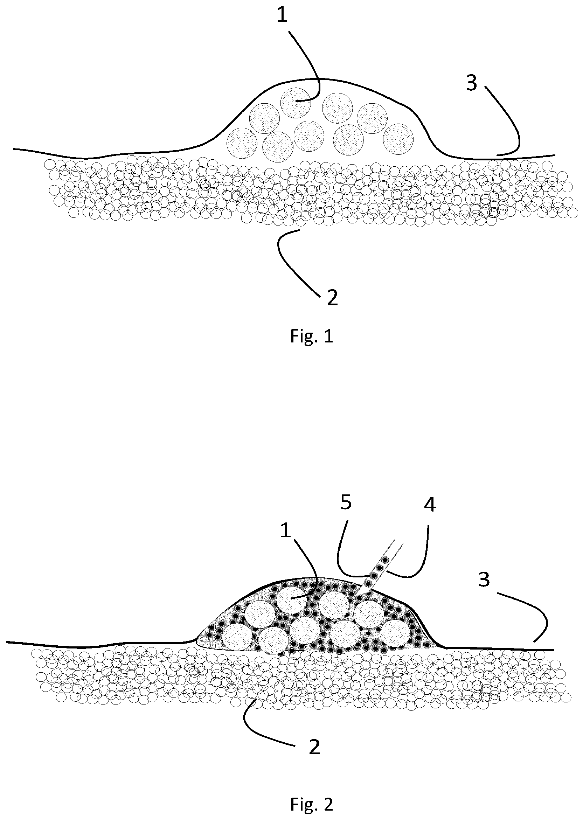

Figure 1 is a representation of a cancerous tumour, containing cancerous cells 1, which are retained by skin or organ membrane 3, with healthy cells 2 beneath.

Figure 2 exemplifies the infusion of the tumour by the injection 4 of coated magnetically susceptible nanoparticles 5 in a suspension. The nanoparticle infused suspension is delivered to the cancerous cells 1 and will fill the interstices between the cells. The healthy cells 2 are too small and close together to allow the suspension between them.

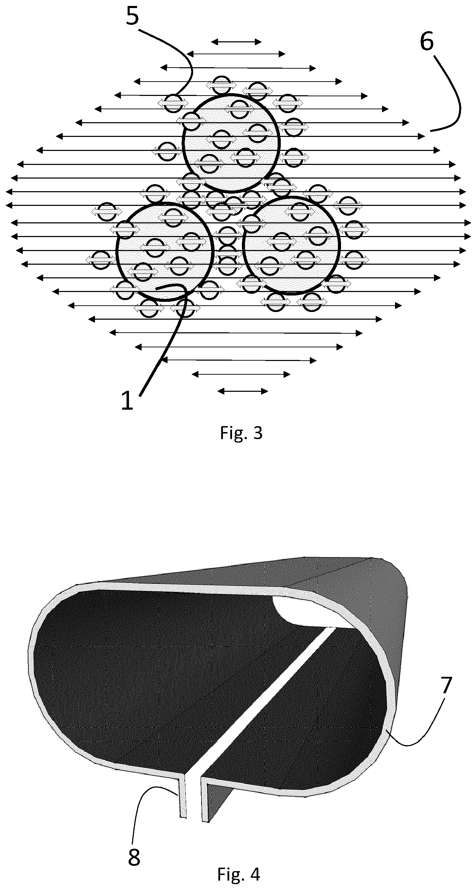

Figure 3 shows the response of the nanoparticles 5 to an oscillating magnetic field 6 in the interstices between the cancerous cells 1. The nanoparticles 5 will oscillate at the frequency of the oscillating magnetic field 6, heating by friction the cancerous cells 1 which, by virtue of less blood flow, are more susceptible to heat than healthy cells. The healthy cells 2, as there are no nanoparticles in their volume, will be minimally affected. The frequency used must not be higher than the sub-megahertz range or the nanoparticles cannot follow the oscillations of the field.

Figure 4 illustrates the slightly compressed oval or elliptical shape of the inductor 7, which may have flanges 8 and which may be constructed of sheet copper or other low resistance material; in another example this could be Graphene. The treatment volume of the magnetic field created may be homogeneous to a tolerance of +/- 10% but typically +/- 5%. This compressed oval shape may reduce the power requirement when the inductor-capacitor array 21 is employed in the treatment of cancer in human subjects.

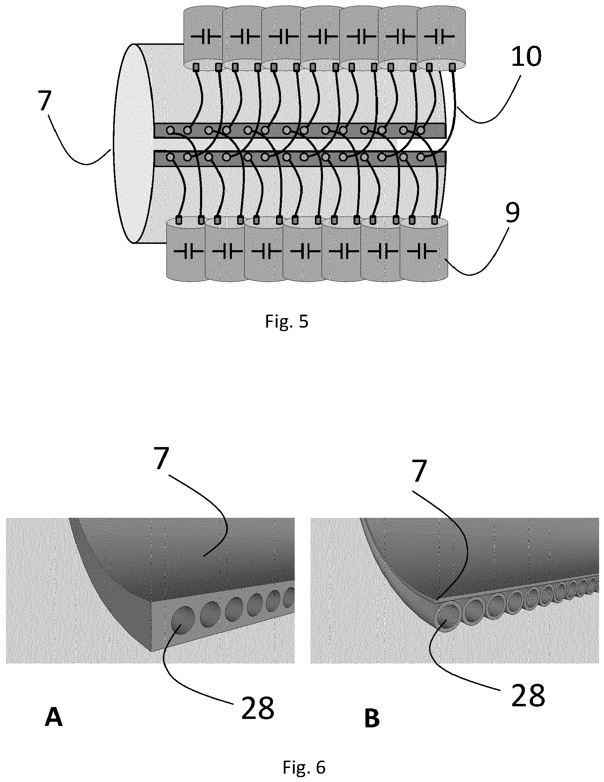

Figure 5 is a representation of the quasi-oval, tubular inductor 7 which may incorporate flanges 8 with an array of parallel high current capacitors 9 attached. The array may be extended longitudinally by the extension of the low reluctance inductor and the addition of further capacitors, forming the resonant circuit. The structural insulation and cooling arrangement are omitted for clarity. The field in this example contains a large volume of magnetic field, in the desired frequency, homogeneous to +/- 10%; this is typically 100 times the sample volume of available state of the art laboratory research equipment.

Figure 6 A & B: as the power requirement for the creation and maintenance of the homogeneous magnetic field is very high, means of cooling the inductor is required.

By way of example two means of construction of the elliptical inductor 7 is described, to include means of cooling, by introduction of a cooling liquid into the structure of the inductor via internal tubes 28A, or pipes 28B thermally connected to the inductor. The active portion of the inductor is less than 2mm or so from the interior surface of the inductor tube, due to electrical high frequency skin effect, so constructions for cooling may be added to the outside of the apparatus without affecting the interior homogeneous magnetic field. In a practical realisation, the cooling fluid may pass between two manifolds, to which the capacitors are also attached, thereby cooling the capacitors.

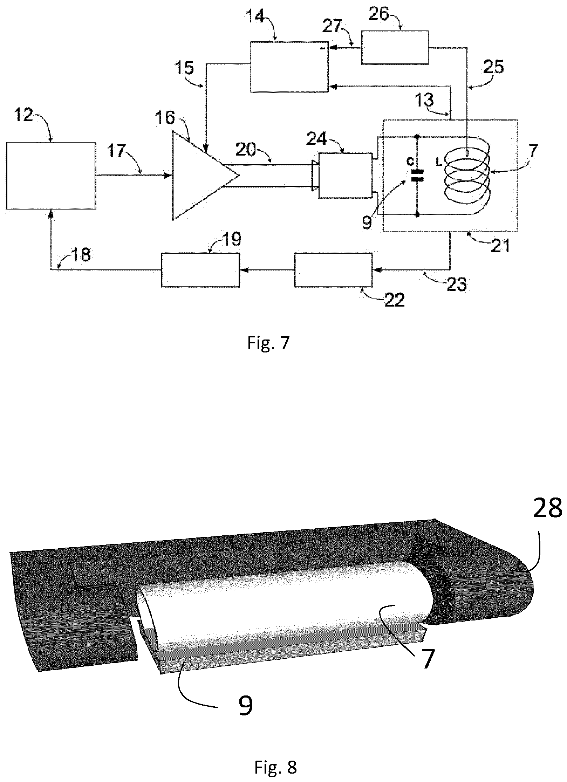

Figure 7 shows an example of the control system of the invention, in which: an adjustable frequency generator 12, produces the required excitation frequency 17, this frequency is then amplified by the power amplifier 16. The high power signal 20 is passed to the matching network 24, which then controls the magnetic field of the resonant circuit 21 formed by the elliptical tube 7 and the parallel capacitors 9.

Three closed loop feedback circuits 13, 25 and 23 are provided:
The first, 13 acting on the field intensity amplifier 16, controls the field intensity. A feedback of the magnetic field intensity 13 via a magnetic field level measurement circuit 14, produces a power control signal 15; the power control signal then modifies the output of the excitation amplifier 16.

The second, also acting on the field intensity amplifier 16 derives from the temperature of the target treatment area 25.

The temperature of the treatment target within the magnetic field transducer 7 is measured via a fibre-optic temperature probe 25. As the temperature of the target approaches the desired value, this value 27 via the signal conditioning circuit 26 takes precedence over the field intensity set-point, and the field level controller 14 will act to reduce the power level control signal 15 such that the required temperature is not exceeded.

The third, resonance optimisation, feedback loop 23 derives the waveform produced by the resonant circuit 21, passing the signal to a digital waveform analysis and mathematical circuit 22 capable of transforming the signal from its original domain to a representation in the frequency domain. This transformed signal may be, but not necessarily, sent to a closed loop control circuit 19, optimised to reduce the total harmonic distortion (THD) in the resonant circuit 21. The frequency correction signal 18 may be passed to the originating signal generator 12, modifying its output.

Figure 8 illustrates the elliptical inductor tube 7 and capacitor array 9. A portion of the space surrounding the cylindrical resonant circuit may consist of a magnetically permeable material 28 to enhance the field density within the apparatus 21 and to aid the containment of stray magnetic field. In large systems, a permeable material structure may reduce the reluctance of the air path external to the cylinder 7, significantly reducing the power requirement for a given flux density in the working volume of homogeneous field.

The elliptical or quasi-oval cylinder 7 and the capacitors 9 are connected in parallel to form a resonant circuit which can resonate at a selectable predetermined frequency, thereby producing a volume of homogeneous magnetic field. The homogeneous field or treatment volume is significantly greater than is possible than that produced by current technology and may be scaled up to accept the human form. This can provide a non-invasive treatment for cancerous tumours, without discomfort to the patient/recipient.

The physical and electrical properties of the cylinder 7 and capacitors 9 are selected such that the predetermined frequency is, by way of example 100 kHz. This is a suitable frequency when using spherical magnetic nanoparticles 2 having a diameter of 5 to 25 nm. For other types or sizes of magnetic particles, a different frequency may be suitable and such adjustment is possible with this invention.

The cylinder 7 can be considered to be a single-turn inductor. However, the inductor has the physical aspect of an elliptical or quasi-oval tube, and can produce a volume of homogeneous magnetic field within it at a lower power requirement than that of a circular cylinder.

Within the internal volume, the magnetic flux density is homogeneous to, typically, better than +/-10% throughout the treatment volume.

For the cylinder 7, the inductance is set by the cylinder geometry. It increases as the diameter increases. However, as the length of the cylinder 7 is increased, the inductance reduces. As an example only, an aspect ratio of between 1 and 3 will result in an inductance which can be brought to resonance in the desired frequency band.

It is desirable to have a resonant circuit with a high Q-factor, to maximise the efficiency of the overall apparatus. In order to achieve this Q-factor, the inductor is fabricated such that its resistance is as low as possible by the choice of materials, a suitable cross-sectional area and, as the voltage and current required by the human scale apparatus is very high, a suitable cooling technique.

The cylinder 7 is formed from copper, or other low resistance material. The cylinder 7 may incorporate a number of tubes in the form of adjacent annular rings 25 A&B which are connected together to form the cylinder.

The annular rings define a number of flow paths for a coolant in the form of water or other coolant to flow through the cylinder 7. The water may be provided at high pressure. An input manifold (not shown) may be provided for directing the water coolant from a source through the flow paths.

An output manifold (not shown) may be provided for collecting the water coolant from the various flow paths.

### A similar arrangement may be formed to cool the capacitors 9

The capacitors 9 may be selected to have very low dielectric losses and minimal contribution to the resistive losses. The excitation is arranged to optimise the dynamic (damping) impedance reflected from the source.

The apparatus 11 is adapted to be used with magnetic nanoparticles (supplied by others) which are inserted into the body of a patient and injected directly into the tumour to occupy the interstices between the cancerous cells.

The magnetic nanoparticles will respond when within the volume of the magnetic field, thereby generating heat. This will selectively heat the tumour, with little effect on healthy tissue, if the magnetic field is alternated at frequencies in the tens to hundreds of kilohertz range.

Therefore, the magnetic field intensity required to produce a local, targeted heating effect is lower than in conventional systems. This allows the apparatus 21 to be scaled up, and even to enclose a human torso, without loss of homogeneity.

A prototype developed by the Applicant has produced a cylindrical homogeneous field volume of 320 mm diameter by 250 mm length. This represents a working volume in the order of 100 times greater than is available with current laboratory research apparatus.

The elliptical cylindrical resonant circuit requires less input energy to produce a given volume of homogeneous magnetic field, compared to other methods. It is compatible with available high power capacitive technology and is easily scaled up in volume.

Disturbing influences, such as temperature changes and the loading effect of the sample within the transducer, can affect the resonant frequency of the parallel-tuned magnetic field transducer circuit.

Another aspect of the present invention is the use of a control system figure 7 to constantly monitor and tune the resonant circuit to maintain the optimum resonance.

Continuous fast amplitude optimisation is used to stabilise the magnetic field against these disturbing influences. A stable field intensity allows a predictable and controlled temperature rise of the nanoparticles.

The control system is shown in Figure 7. The control system includes a signal generator device 12 adapted to generate an excitation frequency for resonating the inductor-capacitor array 21. The generated excitation frequency of the signal generator device 12 is adjustable. The excitation frequency signal is passed through a variable high-power excitation amplifier 16 to provide the required high excitation energy before being fed to the resonant circuit 21.

The control system also includes circuits 19 22 for analysing the waveform of the resonating circuit and determining a level of total harmonic distortion (THD) within the waveform. The device 22 samples the waveform and carries out a Fast Fourier Transform (FFT) to determine the value of the total harmonic distortion within the waveform.

This THD value is passed to a processor device 19 which can determine a suitable excitation frequency of the signal generator device 12 to reduce the determined value of THD. The excitation frequency of the signal generator device 12 is adjusted accordingly.

It should be noted that in another advantage of the invention, the control system provides closed loop control and can rapidly tune the excitation frequency in real time during a treatment, and that minimising the harmonic distortion to the waveform also reduces EMC emissions from the apparatus at the harmonics of the operating frequency.

It is also desirable to adjust the amplitude of the generated signal to maintain stability of the magnetic field.

The magnetic field density can be determined from the geometry of the cylinder 7 and the current flowing through it. The current can be determined from the ratio of the voltage across the cylinder 7 and the impedance of the cylinder.

The inductance of the cylinder 7 is known from the value of capacitance 9 and measurement of the resonant frequency, thus the inductance required to resonate the known capacitance at the actual operating frequency is known. This compensates for the modification of the nominal inductance of the cylinder, due to metallic or permeable materials in the vicinity, or the loading effect of the target within the cylinder 7.

This control system has the flexibility to operate over a wide range of operating frequencies, which correspond to different arrays of capacitance. The system also allows continuous adjustment of the field intensity, from zero to the maximum power level limitation of the excitation system. This flexibility enables optimal operation with varied sizes and compositions of nanoparticles, coatings and fluids.

In summary, there is provided an apparatus configured to generate an oscillating magnetic field having a frequency of between 2 kilohertz and 1 megahertz in a target region, the target region defining a length direction. The apparatus comprises: a sheet conductor (7) extending in the length direction and around the target region to provide a wall of the target region surrounding at least 75% of a cross-section through the target region, the cross-section transverse to the length direction, and the sheet conductor having a first end separated from a second end thereof, the first end and the second end extending in the length direction and defining a separation region therebetween; and one or more capacitors (9) electrically connected to the sheet conductor at the first end and the second end, and across the separation region. The sheet conductor is configured to function as an inductor and, in combination with the one or more capacitors, to form a tank circuit, to thereby generate an oscillating magnetic field in the target region when the tank circuit is driven at a resonant frequency of the tank circuit.

Throughout the description and claims of this specification, the words "comprise" and "contain" and variations of them mean "including but not limited to", and they are not intended to and do not exclude other components, integers, or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, or groups described in conjunction with a particular aspect, embodiment, or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

### Clauses

1. An apparatus configured to generate an oscillating magnetic field having a frequency of between 2 kilohertz and 1 megahertz in a target region, the target region defining a length direction, the apparatus comprising:
   a sheet conductor extending in the length direction and around the target region to provide a wall of the target region surrounding at least 75% of a cross-section through the target region, the cross-section transverse to the length direction, and the sheet conductor having a first end separated from a second end thereof, the first end and the second end extending in the length direction and defining a separation region therebetween; and
   one or more capacitors, distinct from the sheet conductor, and electrically connected to the sheet conductor at the first end and the second end, and across the separation region,
   wherein the sheet conductor is configured to function as an inductor and, in combination with the one or more capacitors, to form a tank circuit, to thereby generate an oscillating magnetic field in the target region when the tank circuit is driven at a resonant frequency of the tank circuit.
2. The apparatus of clause 1, wherein the wall defines a first internal surface normal, having a component directed away from the first end and the second end, and a second internal surface normal, having a first component directed away from the first end and the second end, and a second component perpendicular to the first component and directed towards the first internal surface normal.
3. The apparatus of clause 1 or clause 2, wherein a distance between the first end and the second end across the separation region is less than 10 centimetres.
4. The apparatus of any preceding clause, wherein the sheet conductor defines an internal surface facing into the target region and an external surface opposite the internal surface, and wherein the one or more capacitors are electrically connected to the sheet conductor at the internal surface at the first end and at the internal surface at the second end.
5. The apparatus of any preceding clause, wherein the one or more capacitors are a plurality of capacitors, the plurality of capacitors connected to the sheet conductor at the first end and the second end, at a plurality of different lengthwise locations.
6. The apparatus of clause 5, wherein the plurality of capacitors are at least ten capacitors, together connected to the sheet conductor at at least ten different lengthwise locations.
7. The apparatus of any preceding clause, wherein the one or more capacitors are one or more high current capacitors.
8. The apparatus of any preceding clause, wherein the cross-section through the target region defines a substantially curved shape, having a radius of curvature for any concave regions with respect to the target region of greater than five centimetres.
9. The apparatus of any preceding clause, wherein the sheet conductor comprises a first planar extension at the first end, extending away from the target region, and a second planar extension at the second end, extending parallel to and separated from the first planar extension, the separation region defined between the first planar extension and the second planar extension.
10. The apparatus of any preceding clause, wherein a minimum extent of the target region in a direction transverse to the length direction is greater than 100 millimetres.
11. The apparatus of any preceding clause, wherein the length of the target region is greater than 100 millimetres.
12. The apparatus of any preceding clause, wherein the apparatus is configured to generate the oscillating magnetic field in the target region having a substantially homogeneous field strength throughout the target region.
13. The apparatus of any preceding clause, wherein the apparatus is configured to, when organic tissue is provided in the target region, the organic tissue including nanoparticles comprising a ferromagnetic material, cause heating of the organic tissue in the regions having high concentrations of the nanoparticles, in response to providing the alternating current supply signal to the sheet conductor.
14. The apparatus of any preceding clause, further comprising a cooler for cooling at least one of: the one or more capacitors; and the sheet conductor.
15. The apparatus of any preceding clause, further comprising a magnetically permeable portion, having a magnetic permeability greater than air.
16. The apparatus of any preceding clause, further comprising a temperature sensor configured to output a temperature signal indicative of a temperature within the target region.
17. The apparatus of any preceding clause, further comprising a controller configured to control at least one of a frequency and an amplitude of a supply signal for driving the tank circuit to thereby control at least one of a magnetic field strength and a temperature within the target region.
18. A method of heating a body, the method comprising:
   providing the body within the target region of an apparatus according to any preceding claim; and
   when the body is within the target region, operating the apparatus to generate an oscillating magnetic field in the target region to thereby heat one or more portions of the body having nanoparticles therein, the nanoparticles comprising a ferromagnetic material.
19. The method of clause 18, further comprising supplying the nanoparticles to the body prior to providing the body within the target region.

## Claims

1. An apparatus configured to generate an oscillating magnetic field having a frequency of between 2 kilohertz and 1 megahertz in a target region, the target region defining a length direction, the apparatus comprising:
a sheet conductor extending in the length direction and around the target region to provide a wall of the target region surrounding at least 75% of a cross-section through the target region, the cross-section transverse to the length direction, and the sheet conductor having a first end separated from a second end thereof, the first end and the second end extending in the length direction and defining a separation region therebetween; and
one or more capacitors, distinct from the sheet conductor, and electrically connected to the sheet conductor at the first end and the second end, and across the separation region,
wherein the sheet conductor is configured to function as an inductor and, in combination with the one or more capacitors, to form a tank circuit, to thereby generate an oscillating magnetic field in the target region when the tank circuit is driven at a resonant frequency of the tank circuit.

2. The apparatus as claimed in claim 1, wherein the wall defines a first internal surface normal, having a component directed away from the first end and the second end, and a second internal surface normal, having a first component directed away from the first end and the second end, and a second component perpendicular to the first component and directed towards the first internal surface normal.

3. The apparatus as claimed in claim 1 or claim 2, wherein a distance between the first end and the second end across the separation region is less than 10 centimetres.

4. The apparatus as claimed in any preceding claim, wherein the sheet conductor defines an internal surface facing into the target region and an external surface opposite the internal surface, and wherein the one or more capacitors are electrically connected to the sheet conductor at the internal surface at the first end and at the internal surface at the second end.

5. The apparatus as claimed in any preceding claim, wherein the one or more capacitors are a plurality of capacitors, the plurality of capacitors connected to the sheet conductor at the first end and the second end, at a plurality of different lengthwise locations, optionally wherein the plurality of capacitors are at least ten capacitors, together connected to the sheet conductor at at least ten different lengthwise locations.

6. The apparatus as claimed in any preceding claim, wherein the one or more capacitors are one or more high current capacitors.

7. The apparatus as claimed in any preceding claim, wherein the cross-section through the target region defines a substantially curved shape, having a radius of curvature for any concave regions with respect to the target region of greater than five centimetres, alternatively or additionally wherein the sheet conductor comprises a first planar extension at the first end, extending away from the target region, and a second planar extension at the second end, extending parallel to and separated from the first planar extension, the separation region defined between the first planar extension and the second planar extension.

8. The apparatus as claimed in any preceding claim, wherein a minimum extent of the target region in a direction transverse to the length direction is greater than 100 millimetres, alternatively or additionally wherein the length of the target region is greater than 100 millimetres.

9. The apparatus as claimed in any preceding claim, wherein the apparatus is configured to generate the oscillating magnetic field in the target region having a substantially homogeneous field strength throughout the target region.

10. The apparatus as claimed in any preceding claim, wherein the apparatus is configured to, when organic tissue is provided in the target region, the organic tissue including nanoparticles comprising a ferromagnetic material, cause heating of the organic tissue in the regions having high concentrations of the nanoparticles, in response to providing the alternating current supply signal to the sheet conductor.

11. The apparatus as claimed in any preceding claim, further comprising a cooler for cooling at least one of: the one or more capacitors; and the sheet conductor.

12. The apparatus as claimed in any preceding claim, further comprising a magnetically permeable portion, having a magnetic permeability greater than air.

13. The apparatus as claimed in any preceding claim, further comprising a temperature sensor configured to output a temperature signal indicative of a temperature within the target region.

14. The apparatus as claimed in any preceding claim, further comprising a controller configured to control at least one of a frequency and an amplitude of a supply signal for driving the tank circuit to thereby control at least one of a magnetic field strength and a temperature within the target region.

15. A method of heating a body, the method comprising:
providing the body within the target region of an apparatus according to any preceding claim; and
when the body is within the target region, operating the apparatus to generate an oscillating magnetic field in the target region to thereby heat one or more portions of the body having nanoparticles therein, the nanoparticles comprising a ferromagnetic material, optionally wherein the method further comprises supplying the nanoparticles to the body prior to providing the body within the target region.
